# EUROPEAN PATENT SPECIFICATION

(11) **EP 2 686 044 B1**
(45) Date of publication and mention of the grant of the patent: **20.05.2015**
(21) Application number: 12708857.3
(22) Date of filing: 16.03.2012
(51) Int. Cl.: A61M 5/31, A61M 5/24

(54) **DRUG DELIVERY DEVICE AND METHOD OF ASSEMBLING THE SAME**
WIRKSTOFFFREISETZUNGSVORRICHTUNG UND MONTAGEVERFAHREN DAFÜR
DISPOSITIF D'ADMINISTRATION DE MÉDICAMENT ET SON PROCÉDÉ D'ASSEMBLAGE

(30) Priority: 17.03.2011 EP 11158645
(43) Date of publication of application: 22.01.2014
(73) Proprietor: Sanofi-Aventis Deutschland GmbH, 65929 Frankfurt am Main (DE)
(72) Inventor: HELMER, Michael, 65926 Frankfurt am Main (DE); MATTHIAS, Claudia, 65926 Frankfurt am Main (DE); MUELLER, Lutz, 65926 Frankfurt am Main (DE); RAAB, Steffen, 65926 Frankfurt am Main (DE); NOBER, Peter, 54597 Rommersheim (DE); SHAHBAZFAR, Reza, 65183 Wiesbaden (DE); SCHÄFER, Benjamin, 35649 Bischoffen (DE); ZEIMETZ, Leo, 64572 Büttelborn (DE)
(86) International application number: PCT/EP2012/054643
(87) International publication number: WO 2012/123565

(56) References cited:
- EP-A2- 0 645 152
- WO-A1-2010/037759
- US-A- 5 226 894
- US-A1- 2010 280 461

## Description

### Field of the Invention

This invention relates to a drive mechanism for a drug delivery device that allows a user to select single or multiple doses of an injectable medicinal product and to dispense the set dosage of the product and to apply said product to a patient, preferably by injection. In particular, the present invention relates to such devices, which are handled by the patients themselves.

### Background and Prior Art

Drug delivery devices allowing for multiple dosing of a required dosage of a liquid medicinal product, such as liquid drugs, and further providing administration of the liquid to a patient, are as such well-known in the art. Generally, such devices have substantially the same purpose as that of an ordinary syringe.

Drug delivery devices of this kind have to meet a number of user specific requirements. For instance in case of those with diabetes, many users will be physically infirm and may also have impaired vision. Therefore, these devices need to be robust in construction, yet easy to use, both in terms of the manipulation of the parts and understanding by a user of its operation. Further, the dose setting must be easy and unambiguous and where the device is to be disposable rather than reducible, the device should be inexpensive to manufacture and easy to dispose. In order to meet these requirements, the number of parts and steps required to assemble the device and an overall number of material types the device is made from have to be kept to a minimum.

Typically, the medicinal product to be administered is provided in a cartridge that has a moveable piston or bung mechanically interacting with a piston rod of a drive mechanism of the drug delivery device. By applying thrust to the piston in distal direction, a certain amount of the medicinal fluid is expelled from the cartridge.

A typical drug delivery device designed as pen-type injector is for instance illustrated in WO 2006/037434 A1. This device comprises a housing and a cartridge holder coupled to a distal end of the housing. While the cartridge holder is adapted to receive a cartridge containing a liquid drug, the housing serves as casing for a drive mechanism to be operably engaged with the cartridge.

With drug delivery devices of disposable type, i.e. devices being designed for single- or one-way use, it is particularly intended to undetachably interconnect housing and cartridge holder. If the medicinal fluid contained in the cartridge is used up, the emptied cartridge and the drug delivery device will have to be disposed rather than reused.

For undetachably interconnecting of cartridge holder and housing or casing numerous fixing or interconnecting methods are generally applicable. In particular, if at least one of the two housing components, cartridge holder or casing comprises a thermoplastic material, a laser welding procedure can be applied for interconnecting said housing components. By means of laser welding, thermal energy is deposited in at least one of the two components leading to a respective local melting and bonding of the particular component.

However, when applying a welding procedure for mutually interconnecting components of such drug delivery devices, it has to be ensured, that the two components to be interconnected are in mechanical contact. Preferably, the at least two components should be subject to a surface pressure across a respective contact area in order to provide a sufficiently stable and robust welded interconnection.

Due to manufacturing and assembly tolerances, it is difficult to provide optimal conditions for such welding procedures, e.g. in form of contacting surfaces abutting against each other under the effect of a pretension. Also, since a welding procedure has to be applied to such regions where two housing components at least partially overlap, a direct mechanical contact of housing portions may require exertion of a radially inwardly directed pressure to the outer housing component. One the one hand, such external pressure exertion is quite elaborate. On the other hand, the respective housing component may become subject to considerable mechanical stress and deformation, which may reduce the durability of the housing component.

Making use of tightly fitting housing components, their respective contact surfaces may get in mutual mechanical contact to a large extent. However, tightly fitting housing components are quite difficult to assemble, e.g. due to corresponding frictional forces arising during a mutual arrangement of housing components into one another.

Document EP 0 645 152 A2 discloses a holder featuring a hollow body for housing a cartridge-needle unit therein. The body is of oval shape and engages with an adapter ring with retaining slots. By squeezing the body inwardly along a major axis of its ellipse, a relative axial movement of body and adapter ring can be provided.

Document WO 2010/037759 A1 refers to a pen-type injector featuring an activation means at a proximal end. Said activation means comprises an activation button operationally connected to a spring locking means which comprises a deformable ring-shaped member having an inner diameter somewhat larger than an annular latch of a pressure member.

Document US 5 226 894 A exhibits an elliptically-shaped body which permits an axial movement with an adapter ring when appropriately squeezed.

Document US 2010/0280461 A1 discloses nested or interleaved arrangement of cartridge holder and proximal housing component, wherein these components can be bonded, e.g. by way of laser welding.

### Object of the Invention

It is therefore an object of the present invention to provide a drug delivery device for dispensing of a dose of a medicinal product, which on the one hand allows for an easy and smooth assembly and which on the other hand provides a sufficiently large contact area and respective contact surfaces that allow for a robust, rigid and stable welding of housing components. The invention also focuses on a respective method of assembly being particularly suitable for mass-production of drug delivery devices. It is a further object of the invention to simplify construction and assembly of a drug delivery device, in particular of its housing components.

### Summary of the Invention

In a first aspect, the invention provides a drug delivery device for dispensing of a dose of a medicinal product, typically a medicinal fluid, such as a fluid drug like insulin or heparin. The drug delivery device comprises a first housing component and a second housing component. Typically, first and second housing components are designed as cartridge holder for a fluid-containing cartridge and/or as a casing for a drive mechanism, which is to be operably engaged with the cartridge, in particular with its displaceable piston once the device has been assembled finally.

Irrespective on whether the first or second housing components are designed as casing or cartridge holder, the first housing component comprises a receptacle being adapted to at least partially receive the second housing component, in particular an insert portion of the second housing component. Hence, first and second housing components are at least partially to be pushed into one another leading to an interleaved or intertwined arrangement of first and second housing components. In this way, an outer surface of the insert portion of the second housing component may abut with an inner surface of the receptacle of the first housing component. Shape and design of receptacle and insert portion therefore define contact surfaces across which an undetachable interconnection of first and second housing components can be established.

Typically, first and second housing components are generally of cylindrical-like but oval shape. Since the insert portion of the second housing component is at least partially insertable into the receptacle of the first housing component along an axial direction, first and second housing components are mutually displaceable along their cylinder long axis.

In order to transfer the drug delivery device and its first and second housing components into a pre-fixing assembly configuration, first and second housing components comprise such dimensions and geometries that support a radially acting mutual clamping of first and second housing components. In particular, the outside dimension or at least one outside diameter of the insert portion of the second housing component is at least in sections larger than a corresponding inner dimension or a respective inner diameter of the receptacle of the first housing component.

Outside and inner dimensions or respective outside and inner diameters of insert portion and receptacle correspond to each other in such a way, that the insert portion can be easily and smoothly inserted into the corresponding receptacle of the first housing component. During or after insertion or pre-assembly, first and second housing component are adapted to become subject to a radially acting clamping, that transfers the drug delivery device and its housing components in a pre-fixing assembly configuration. The pre-fixing assembly configuration is characterized in that respective contact surfaces of insert portion and receptacle are subject to such frictional forces being large enough to prevent autonomous disassembling of the drug delivery device. Also, in said pre-fixing assembly configuration, insert portion and receptacle are preferably subject to a radially acting pretension, thus spatially fixing and immobilizing said first and second housing components during a subsequent final assembly stage, e.g. during an interconnecting welding process.

The insert portion of the second housing component is at least slightly oval- or elliptically shaped in radial cross section. Having a break of symmetry in the radial cross section of the insert portion, a clamping with a correspondingly symmetry broken receptacle can be achieved by rotating first and second housing components in a circumferential direction with respect to each other.

Moreover, also the inner wall of the receptacle is oval- or elliptically shaped in radial cross section. In this way, if long and short axes of insert portion and receptacle are arranged substantially parallel, a smooth running insertion of the insert portion into a receptacle will be executable. When the insert portion has been at least partially inserted into its corresponding receptacle, by mutually rotating first and second housing components in circumferential direction, a radial clamping and a respective built-up of a radially acting pretension can be established.

Accordingly, a mutual clamping and pre-fixing of first and second housing components is producible by means of rotating first and/or second housing components with respect to each other in circumferential direction, after or during insertion of the second housing component's insert portion into the receptacle of the first housing component. Shape and geometry of oval- or elliptically shaped insert portion and receptacle is preferably such, that the insert portion's long axis is larger than the receptacle's short axis. In this way, a mutual rotation of insert portion and receptacle inevitably leads to a radial clamping and to a built-up of a respective pretension between the two housing components.

Depending on the respective geometry of insert portion and receptacle, in said pre-fixing configuration, the insert portion of the second housing component is adapted to exert a radially outwardly directed pretension to the receptacle of the first housing component. Also in the opposite way, it is conceivable, that the receptacle of the first housing component is adapted to exert a radially inwardly directed pretension to the insert portion of the second housing component.

Generally, for the purpose of establishing a pre-fixing by means of clamping, it is sufficient to generate a pretension on the contact surfaces of first and second housing components, respectively.

In another aspect of the invention, the first and second housing components are integrally connected by means of welding and/or by means of an adhesive. Either the receptacle or the insert portion of first and second housing components can be provided with a suitable adhesive before first and second housing components are arranged in an intertwined way. By transferring first and second housing components into a pre-fixing assembly configuration, the two components become spatially fixed and immobilized with respect to each other by means of clamping allowing the adhesive to be cured appropriately. External and/or additional means for keeping first and second housing components in position are therefore no longer required. In this way, the complexity of a mass-production process can be advantageously reduced.

According to another preferred embodiment, first and second housing components are subject to a laser welding procedure, wherein thermal energy is deposited in either first or second housing components by means of electromagnetic radiation, which leads to a local melting of the respective housing material. For the purpose of laser welding, the first housing component comprises at least one portion being at least partially transparent for a particular laser radiation wavelength. Correspondingly, said laser radiation of choice is at least partially absorbable by the second housing component, preferably by its insert portion. In this way, the applied laser radiation can propagate through at least a side wall portion of the first housing component with rather low absorption losses. However, the inner insert portion is adapted to at least partially absorb the applied laser radiation. This absorption process is accompanied by a respective deposition of thermal energy, preferably in the vicinity of the contact surfaces of insert portion and receptacle.

Preferably, the contact surface of the insert portion becomes subject to exposure of electromagnetic radiation and consequently subject to an at least local melting and bonding. Since said contact surface is preferably subject to a radially acting pretension, the melted material may widely distribute, leading to weld portions or weld spots being increased in size compare to configurations, where the contact surfaces are in a mere loose contact.

By having the housing components arranged in a pretensioned pre-fixing assembling configuration, the two components remain inherently fixed to each other during a laser welding procedure. It is further of advantage, that in this way the two housing components do not have to be fixed, e.g. by separate fixing means during such a laser welding. In this way, the overall handling of the pre-fixed drug delivery device during a laser welding process can be simplified and facilitated. Additionally, by means of the pre-fixing assembly configuration, any additional means for bringing respective contact surfaces of receptacle and insert portion in mechanical contact are no longer required.

According to another preferred embodiment of the invention, the first housing component comprises at least one tongue section being elastically deformable in radial direction and being adapted to bear against an inner side wall section of the second housing component, preferably against an inner side wall of said housing component's receptacle. By making use of such flexible deformable clamping elements, a comparable clamping and pretensioning of first and second housing components can be achieved even without the necessity of rotating first and second housing components with respect to each other.

In typical configurations, the at least one tongue section projects radially outwardly in an initial configuration and becomes radially inwardly biased in the course of inserting the second housing component's insert portion into the corresponding receptacle of the first housing component. Making use of such tongue sections, radial clearance or radial gaps between insert portion and receptacle can be effectively eliminated already in the course of the intertwined insertion of first and second housing components, respectively.

In this way it can be further ensured, that the insert portion also gets in direct contact with the inner side wall section of the second housing component at least with its radially projecting tongue section. In a subsequent stage of final assembly, said tongue sections are then preferably subject to laser welding. Hence, a radially outwardly pointing surface of the tongue section getting in direct contact with the inner side wall section of the second housing component preferably becomes subject to laser-induced melting.

Correspondingly and additionally or alternatively, it is also conceivable, that the second housing component comprises at least one tongue section being elastically deformable in radial direction and being adapted to bear against an outer side wall section of the first housing component. This tongue section typically provides a radially inwardly directed pretension during or after the second housing component's insert portion has been placed into the receptacle.

In further embodiments it is even conceivable, that both, receptacle as well as insert portion of first and second housing components comprise elastically deformable tongue sections being for instance alternatively arranged in circumferential direction, respectively.

According to a further preferred embodiment of the invention, the at least one tongue section of either insert portion or receptacle comprises a rounded and/or convex shaped support surface. Such rounded and/or convex shaped support surfaces may act as a run-on slope, facilitating a radially inwardly or radially outwardly directed elastic deformation of respective tongue sections during axial insertion of the insert portion into the corresponding receptacle. Moreover, by means of a rounded and/or convex shaped support surface, mutual abutment of the support surface with a corresponding contact surface of the respective upper housing component can be effectively provided.

According to a further preferred embodiment, at least numerous tongue sections are distributed across the circumference of first and/or second housing components. Particularly, the tongue sections extend in axial direction and are preferably evenly distributed across the circumference of the second housing component's insert portion and/or at the inner side wall of the first housing component's receptacle.

According to a further independent aspect, the invention also relates to a method of assembling a drug delivery device comprising a first housing component having a receptacle being oval - or elliptically shaped in radial cross section and a second housing component having an insert portion being oval- or elliptically shaped in radial cross section and being at least partially insertable into said first housing component along an axial direction. For assembling said drug delivery device, the insert portion and the receptacle of first and second housing components are at least partially inserted into one another in an axial direction. During or after this insertion, a radially directed pretension is built-up and generated between first and second housing components in order to achieve a respective pre-fixing clamping for mutually interlocking of first and second housing components in a respective pre-fixing configuration.

Thereafter, first and second housing components are undetachably interconnected by means of a laser welding procedure. Preferably, said laser welding is particularly applied to such surface portions of first and second housing components being in direct mutual contact.

In a further preferred embodiment, first and second housing components comprise an insert portion and a corresponding receptacle, wherein the outside dimension or an at least one outside diameter of the insert portion is at least in sections larger than a corresponding inner dimension of the receptacle. Due to this dimensions and geometries of first and second housing components and their respective insert portion and receptacle, a pre-fixed clamped and pretensioned configuration can be established by means of rotating first and second housing components with respect to each other in a circumferential direction.

If designed as radially acting deformable tongue portions, the clamping means can also establish a clamped configuration of first and second housing components even without relative circumferential rotation.

Hence, additional fixing or mounting means for immobilizing first and second housing components during a laser welding procedure may become superfluous.

Also, by way of generating a radially acting pretension, the quality and durability of a laser welded interconnection can be enhanced advantageously.

It will be apparent to those skilled in the pertinent art, that various modifications and variations can be made to the present invention without departing from the spirit and scope of the invention. Further, it is to be noted, that any reference signs used in the appended claims are not to be construed as limiting the scope of the present invention.

### Brief Description of the Drawings

Without limitation, the present invention will be explained in greater detail below in connection with preferred embodiments and with reference to the drawings in which:
- Figure 1: schematically illustrates two housing components of a drug delivery device being oval or elliptic in cross-section,
- Figure 2: separately shows the two housing components in radial cross section,
- Figure 3: schematically shows an axial or longitudinal cross section of first and second housing components in an assembly configuration,
- Figure 4: depicts the housing components in unlocked and locked configuration,
- Figure 5: shows a cross sectional illustration of a tongue section in initial configuration and
- Figure 6: illustrates the tongue section according to Figure 5 in a pre-stressed and pretensioned configuration.

### Detailed Description

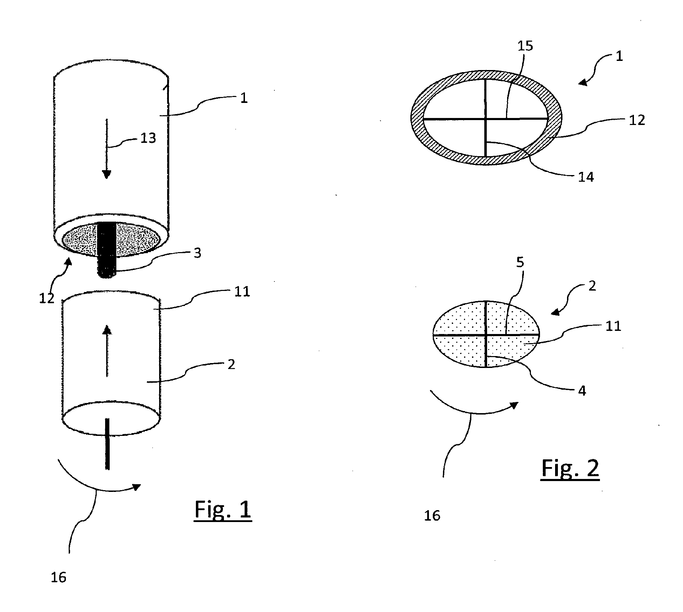

The illustrations according to Figures 1 and 2 exemplary illustrates first and second housing components 1, 2 of a not further shown drug delivery device. In this embodiment, the first housing component 1 is designed as a casing for a not particularly illustrated drive mechanism of the drug delivery device. Only a piston rod or drive sleeve 3 to be operably engaged with a pressure piece or with a piston 6 of a cartridge 9 is shown. The second housing component 2 is designed as a cartridge holder and is therefore adapted to receive a cartridge 9 containing the fluid drug to be disposed by the drug delivery device, typically a pen-type injector.

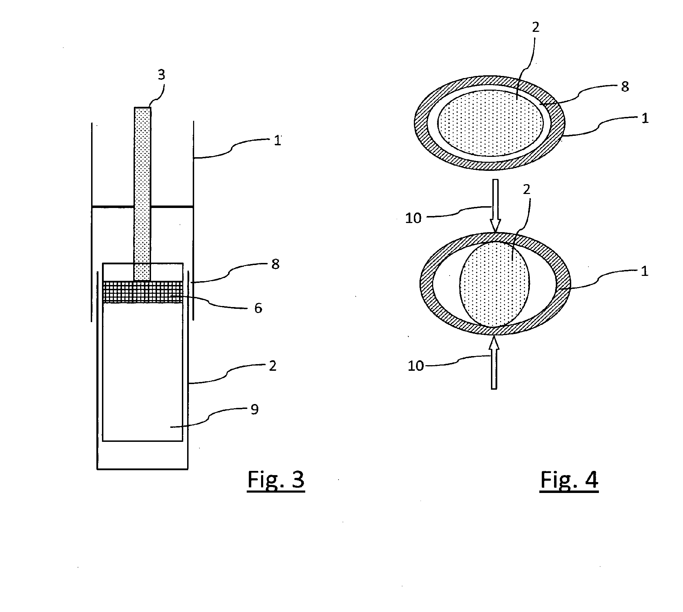

The two housing components 1, 2 comprise an oval- or elliptically shaped radial cross section as illustrated in the two separate sketches of Figure 2. Initially, the two housing components 1, 2 are arranged and disposed in such a way, that their respective long axes 5, 15 and short axes 4, 14 are substantially arranged in parallel. In such an initial configuration as illustrated in the upper sketch of Figure 4, the second housing component 2 can be at least partially inserted into the first housing component 1 along the axial direction 13.

In particular, the second housing component 2 can be inserted into the receptacle 12 of the first housing component with an insert portion 11. As soon as the two housing components 1, 2 are arranged in an intertwined or interleaved manner, as illustrated in the upper sketch of Figure 4, by rotating first and/or second housing components 1, 2 with respect to each other in circumferential direction 16, a radial clamping of first and second housing components 1, 2 can be established as it is illustrated in the lower sketch of Figure 4. Consequently, a radially inwardly or radially outwardly directed pretension 10 may act on the insert portion 11 or on the side walls of the receptacle 12 of respective second and/or first housing components 2, 1.

The geometry and dimensions of receptacle 12 and insert portion 11 is chosen such, that the long axis 5 of the insert portion is larger than the short axis 14 of the receptacle 12. Generally, the insert portion's long axis 5 is smaller than the receptacle's long axis 15. Also, the insert portion's short axis 4 is smaller or shorter than the respective short axis 14 of the receptacle 12 of the first housing component 1.

Since insert portion 11 and receptacle 12 are similar in shape, a radial gap 8 is formed between insert portion 11 and receptacle 12 upon their at least partial interleaved displacement. By means of this radial gap 8, the insert portion 11 can be smoothly inserted into the corresponding receptacle 12. Eventual friction forces can be kept in a rather low range. As soon as a final assembling position in axial direction has been reached, the two housing components 1, 2 can be axially fixed and interlocked with respect to each other simply by rotating and turning first and second housing components 1, 2 with respect to each other in circumferential direction 16.

In a subsequent laser welding process, first and second housing components 1, 2 are preferably welded in the region, where insert portion 11 and receptacle 12 get in direct contact when reaching the pre-fixing assembly configuration as depicted in the lower sketch of Figure 4.

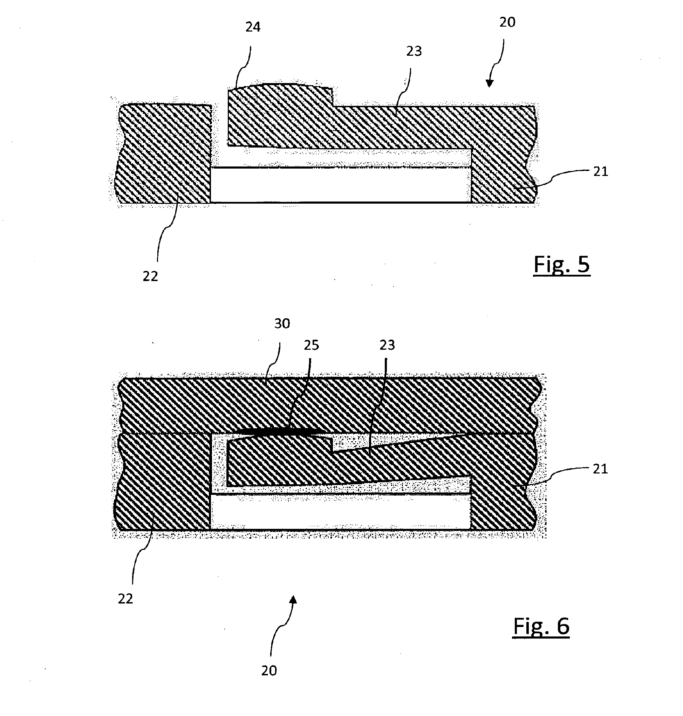

In Figures 5 and 6, another embodiment of the invention is illustrated, wherein the first housing component 20 comprises two housing sections 21, 22, wherein the housing section 21 is integrally formed with an elastically deformable tongue portion 23. In Figure 5, the housing component 20 is illustrated in an initial configuration, wherein the tongue portion 23 at least slightly extends from the housing section 22 in radial direction with its contact surface 24. In the illustrations according to Figures 5 and 6, the radial direction is oriented in vertical direction and the axial direction points substantially horizontally.

As soon as the housing component 20 is axially inserted into a second housing component 30 having a receptacle with an inner side wall, the tongue section 23 with its contact surface 24 becomes subject to a radially inwardly directed elastic bending. Due to the material inherent reset force, the tongue section 23 exerts radially outwardly directed pre-stress to the inner surface of the sidewall of the second housing component 30. This pretension serves to pre-fix first and second housing components 20, 30 with respect to each other and to enhance the quality of the subsequent laser welding process.

In Figure 6, a laser-induced weld spot 25 is indicated. For the purpose of laser welding, the outer housing component 30 is substantially transparent for the laser radiation of choice, whereas at least the tongue section 23 in the area of its contact surface 24 absorbs that laser radiation and therefore becomes subject to thermal energy deposition. In the course of thermal heating by laser radiation, the contact surface 24 of the tongue section 23 becomes subject to melting, which leads to a welded bonding of first and second housing components 20, 30. Since the tongue section 23 exerts radially outwardly directed pre-stress to the corresponding side wall section of the housing component 30, the melted material of the contact surface 24 distributes over the contact surface, thus leading to an effective increase of a weld spot 25.

By means of the rounded or convex shaped contact surface 24, the elastically deformable tongue section 23 comprises a sufficiently large contact area adapted to abut against the sidewall of the housing component 30. Moreover, a rounded or convex-shaped contact surface 24 may also serve as a run-on slope or as a bevelled surface, facilitating mutual insertion of first and housing components 20, 30 in axial direction.

### List of Reference Numerals

- 1: housing component
- 2: housing component
- 3: piston rod
- 4: short axis
- 5: long axis
- 6: pressure piece
- 8: gap
- 9: cartridge
- 10: radial tension
- 11: insert portion
- 12: receptacle
- 13: axial direction
- 14: short axis
- 15: long axis
- 16: circumferential direction
- 20: housing component
- 21: housing section
- 22: housing section
- 23: tongue section
- 24: contact surface
- 25: weld spot
- 30: housing component

## Claims

1. Drug delivery device for dispensing of a dose of a medicinal product, comprising:
- a first housing component (1; 30) comprising a receptacle (12) adapted to at least partially receive
- an insert portion (11) of a second housing component (2; 20) being at least partially insertable into said first housing component (1; 30) along an axial direction (13),
- wherein the outside dimension or an at least one outside diameter of the insert portion (11) is at least in sections larger than a corresponding inner dimension or an at least one inner diameter of the receptacle (12) and wherein insert portion (11) and receptacle (12) are adapted to generate a radially acting mutual clamping of first and second housing components (1, 2; 20, 30) in a pre-fixing assembly configuration, **characterized in that**:
- the receptacle (12) is oval- or elliptically shaped in radial cross section and and **in that** the insert portion (11) is oval- or elliptically shaped in radial cross section, and
- wherein a mutual clamping and pre-fixing of first and second housing components (1, 2; 30, 20) is producible by means of rotating first and/or second housing components (1, 2; 30, 20) with respect to each other in a circumferential direction (16).

2. Drug delivery device according to any one of the preceding claims, wherein in said pre-fixing configuration, the insert portion (11) of the second housing component (2; 20) is adapted to exert radially outwardly directed pretension (10) to the receptacle (12) of the first housing component (1; 30) and/or wherein the receptacle (12) of the first housing component (1; 30) is adapted to exert radially inwardly directed pretension (10) to the insert portion (11) of the second housing component (2; 20).

3. Drug delivery device according to any one of the preceding claims, wherein first and second housing components (1, 2; 30, 20) are integrally connected by means of welding and/or by means of an adhesive.

4. Drug delivery device according to any one of the preceding claims, wherein the first housing component (1; 30) comprises at least one portion being at least partially transparent for a laser radiation being at least partially absorbable by the second housing component (2; 20).

5. Drug delivery device according to any one of the preceding claims, wherein the first and/or second housing component (30, 20) comprises at least one tongue section (23) being elastically deformable in radial direction and being adapted to bear against an inner or outer side wall section of the second or first housing component (20, 30).

6. Drug delivery device according to any one of the preceding claims, wherein the second housing component (20) comprises at least one tongue section being elastically deformable in radial direction and being adapted to bear against an outer side wall section of the first housing component (30).

7. Drug delivery device according to any one of the preceding claims 5 or 6, wherein the at least one tongue section (23) comprises a rounded and/or convex shaped support surface (24).

8. Drug delivery device according to any one of the preceding claims 5 to 7, wherein numerous tongue sections (23) are distributed across the circumference of first and/or second housing components (1, 2; 30, 20).

9. Drug delivery device according to any one of the preceding claims, wherein first and second housing components (1, 2; 30, 20) are designed as cartridge holder for a product-containing cartridge (9) and/or as a casing for a drive mechanism to be operably engaged with the cartridge.

10. A method of assembling a drug delivery device comprising a first housing component (1; 30) having a receptacle (12) being oval - or elliptically shaped in radial cross section and a second housing component (2; 20) having an insert portion (11) being oval- or elliptically shaped in radial cross section and being at least partially insertable into said first housing component (1; 30) along an axial direction (13), the method comprising the steps of:
- at least partially inserting the insert portion (11) and the receptacle (12) of first and second housing components (1, 2; 30, 20) into one another in an axial direction (13) and generating a radially directed pretension (10) between first and second housing components (1, 2; 30, 20) for mutually interlocking of first and second housing components (1, 2; 30, 20) in a pre-fixing configuration,
- undetachably interconnecting first and second housing components (1, 2; 30, 20) by means of laser welding.

11. The method according to claim 10, wherein first and second housing components (1, 2; 30, 20) comprise an insert portion (11) and a corresponding receptacle (12), wherein the outside dimension or an at least one outside diameter of the insert portion (11) is at least in sections larger than a corresponding inner dimension of the receptacle (12) and wherein first and second housing components (1,2; 30, 20) become mutually pre-tensioned in radial direction by means of rotating first and second housing components (1, 2; 30, 20) with respect to each other in a circumferential direction (16).

## Patentansprüche

1. Medikamenten-Verabreichungsvorrichtung zur Abgabe einer Dosis eines Medizinprodukts, umfassend:
- eine erste Gehäusekomponente (1; 30) mit einem Aufnahmebehälter (12) zur zumindest teilweisen Aufnahme
- eines Einsatzabschnitts (11) einer zweiten Gehäusekomponente (2; 20), der zumindest teilweise in die erste Gehäusekomponente (1; 30) entlang einer axialen Richtung (13) eingeführt werden kann,
- worin die Außenabmessung oder zumindest ein Außendurchmesser des Einsatzabschnitts (11) zumindest in Abschnitten größer als eine entsprechende Innenabmessung oder zumindest ein Innendurchmesser des Aufnahmebehälters (12) ist und worin der Einsatzabschnitt (11) und der Aufnahmebehälter (12) ausgelegt sind, eine radial wirkende gegenseitige Klemmung der ersten und zweiten Gehäusekomponenten (1, 2; 20, 30) in einer vorfixierten Zusammenbaukonfiguration zu erzeugen,
**dadurch gekennzeichnet, dass**:
- der Aufnahmebehälter (12) im radialen Querschnitt oval oder elliptisch geformt ist und dass der Einsatzabschnitt (11) im radialen Querschnitt oval oder elliptisch geformt ist, und
- worin eine gegenseitige Klemmung und Vorfixierung der ersten und zweiten Gehäusekomponenten (1, 2; 30, 20) durch Drehen der ersten und/oder zweiten Gehäusekomponenten (1, 2; 30, 20) relativ zueinander in eine Umfangsrichtung (16) erzeugt werden kann.

2. Medikamenten-Verabreichungsvorrichtung nach Anspruch 1,
worin der Einsatzabschnitt (11) der zweiten Gehäusekomponente (2; 20) in der vorfixierten Zusammenbaukonfiguration dazu ausgelegt ist, eine radial auswärts gerichtete Vorspannung (10) auf den Aufnahmebehälter (12) der ersten Gehäusekomponente (1; 30) auszuüben, und/oder worin der Aufnahmebehälter (12) der ersten Gehäusekomponente (1; 30) ausgelegt ist, eine radial einwärts gerichtete Vorspannung (10) auf den Einsatzabschnitt (11) der zweiten Gehäusekomponente (2; 20) auszuüben.

3. Medikamenten-Verabreichungsvorrichtung nach einem der vorhergehenden Ansprüche,
worin erste und zweite Gehäusekomponenten (1, 2; 30, 20) durch Schweißen und/oder mit einem Klebstoff einstückig miteinander verbunden sind.

4. Medikamenten-Verabreichungsvorrichtung nach einem der vorhergehenden Ansprüche,
worin die erste Gehäusekomponente (1; 30) zumindest einen Abschnitt umfasst, der zumindest teilweise für Laserstrahlung transparent ist, die zumindest teilweise von der zweiten Gehäusekomponente (2; 20) absorbiert werden kann.

5. Medikamenten-Verabreichungsvorrichtung nach einem der vorhergehenden Ansprüche,
worin die erste und/oder zweite Gehäusekomponente (30, 20) zumindest einen Zungenabschnitt (23) umfasst, der in radialer Richtung elastisch verformbar ist und ausgelegt ist, an einem inneren oder äußeren Seitenwandabschnitt der zweiten oder ersten Gehäusekomponente (20, 30) anzuliegen.

6. Medikamenten-Verabreichungsvorrichtung nach einem der vorhergehenden Ansprüche,
worin die zweite Gehäusekomponente (20) zumindest einen Zungenabschnitt umfasst, der in radialer Richtung elastisch verformbar ist und ausgelegt ist, an einem äußeren Seitenwandabschnitt der ersten Gehäusekomponente (30) anzuliegen.

7. Medikamenten-Verabreichungsvorrichtung nach einem der vorhergehenden Ansprüche 5 oder 6,
worin der zumindest eine Zungenabschnitt (23) eine abgerundete und/oder konvex geformte Stützfläche (24) umfasst.

8. Medikamenten-Verabreichungsvorrichtung nach einem der vorhergehenden Ansprüche 5 bis 7,
worin zahlreiche Zungenabschnitte (23) über den Umfang der ersten und/oder zweiten Gehäusekomponenten (1, 2; 30, 20) verteilt sind.

9. Medikamenten-Verabreichungsvorrichtung nach einem der vorhergehenden Ansprüche,
worin erste und zweite Gehäusekomponenten (1, 2; 30, 20) als Kartuschenhalter für eine, ein Präparat enthaltende Kartusche (9) und/oder als Gehäuse für einen Antriebsmechanismus, der mit der Kartusche in Wirkeingriff gebracht werden kann, konstruiert sind.

10. Verfahren zur Montage einer Medikamenten-Verabreichungsvorrichtung, die eine erste Gehäusekomponente (1; 30) mit einem Aufnahmebehälter (12), der im radialen Querschnitt oval oder elliptisch geformt ist, und eine zweite Gehäusekomponente (2; 20) mit einem Einsatzabschnitt (11) umfasst, der im radialen Querschnitt oval oder elliptisch geformt ist und zumindest teilweise in die erste Gehäusekomponente (1; 30) entlang einer axialen Richtung (13) eingeführt werden kann, wobei das Verfahren die folgenden Schritte umfasst:
- zumindest teilweises Einführen des Einsatzabschnitts (11) und des Aufnahmebehälters (12) der ersten und zweiten Gehäusekomponenten (1, 2; 30, 20) ineinander in einer axialen Richtung (13) und Erzeugen einer radial gerichteten Vorspannung (10) zwischen den ersten und zweiten Gehäusekomponenten (1, 2; 30, 20) zur gegenseitigen Verriegelung der ersten und zweiten Gehäusekomponenten (1, 2; 30, 20) in einer vorfixierten Konfiguration,
- unlösbares Verbinden der ersten und zweiten Gehäusekomponenten (1, 2; 30, 20) durch Laserschweißen.

11. Verfahren nach Anspruch 10, worin die ersten und zweiten Gehäusekomponenten (1, 2; 30, 20) einen Einsatzabschnitt (11) und einen entsprechenden Aufnahmebehälter (12) umfassen, worin die Außenabmessung oder zumindest ein Außendurchmesser des Einsatzabschnitts (11) zumindest in Abschnitten größer als eine entsprechende Innenabmessung des Aufnahmebehälters (12) ist und worin erste und zweite Gehäusekomponenten (1, 2; 30, 20) durch Drehen der ersten und zweiten Gehäusekomponenten (1, 2; 30, 20) relativ zueinander in eine Umfangsrichtung (16) in radialer Richtung gegenseitig vorgespannt werden.

## Revendications

1. Dispositif d'administration de médicament servant à distribuer une dose d'un produit médicinal, comprenant :
- un premier composant de logement (1 ; 30) comprenant un réceptacle (12) conçu pour recevoir au moins partiellement
- une partie emboîtable (11) d'un second composant de logement (2 ; 20) qui peut être au moins partiellement emboîté dans ledit premier composant de logement (1 ; 30) le long d'une direction axiale (13),
- la dimension extérieure ou au moins un diamètre extérieur de la partie emboîtable (11) étant, au moins au niveau de certaines sections, supérieur(e) à une dimension intérieure ou au moins un diamètre intérieur correspondant du réceptacle (12) et la partie emboîtable (11) et le réceptacle (12) étant conçus pour générer une force de fixation mutuelle des premier et second composants de logement (1, 2 ; 20, 30) agissant radialement dans une configuration d'assemblage pré-assujettissement, **caractérisé en ce que** :
- le réceptacle (12) est de forme ovale ou elliptique en coupe radiale et **en ce que** la partie emboîtable (11) est de forme ovale ou elliptique en coupe radiale, et
- une force de serrage mutuelle et de pré-assujettissement des premier et second composants de logement (1, 2 ; 30, 20) pouvant être produite par le biais de la rotation du premier et/ou du second composant de logement (1, 2 ; 30, 20) l'un par rapport à l'autre dans une direction circonférentielle (16).

2. Dispositif d'administration de médicament selon la revendication 1, dans lequel, dans ladite configuration pré-assujettissement, la partie emboîtable (11) du second composant de logement (2 ; 20) est conçue pour exercer une force de pré-tension (10) dirigée radialement vers l'extérieur sur le réceptacle (12) du premier composant de logement (1 ; 30) et/ou dans lequel le réceptacle (12) du premier composant de logement (1 ; 30) est conçu pour exercer une force de pré-tension (10) dirigée radialement vers l'intérieur sur la partie emboîtable (11) du second composant de logement (2 ; 20).

3. Dispositif d'administration de médicament selon l'une quelconque des revendications précédentes, dans lequel les premier et second composants de logement (1, 2 ; 30, 20) sont raccordés de façon à constituer une seule pièce par soudage et/ou au moyen d'un adhésif.

4. Dispositif d'administration de médicament selon l'une quelconque des revendications précédentes, dans lequel le premier composant de logement (1 ; 30) comprend au moins une partie qui est au moins partiellement perméable à un rayonnement laser qui est au moins partiellement absorbable par le second composant de logement (2 ; 20).

5. Dispositif d'administration de médicament selon l'une quelconque des revendications précédentes, dans lequel le premier composant de logement et/ou le second composant de logement (30, 20) comprennent au moins une section formant languette (23) qui est déformable élastiquement dans la direction radiale et qui est conçue pour être en appui contre une section formant paroi latérale intérieure ou extérieure du second ou du premier composant de logement (20, 30).

6. Dispositif d'administration de médicament selon l'une quelconque des revendications précédentes, dans lequel le second composant de logement (20) comprend au moins une section formant languette qui est déformable élastiquement dans la direction radiale et qui est conçue pour être en appui contre une section formant paroi latérale extérieure du premier composant de logement (30).

7. Dispositif d'administration de médicament selon l'une quelconque des revendications précédentes 5 et 6, dans lequel la ou les sections formant languettes (23) comprennent une surface de support (24) de forme arrondie et/ou convexe.

8. Dispositif d'administration de médicament selon l'une quelconque des revendications précédentes 5 à 7, dans lequel de multiples sections formant languettes (23) sont réparties sur l'ensemble de la circonférence du premier et/ou du second composant de logement (1, 2 ; 30, 20).

9. Dispositif d'administration de médicament selon l'une quelconque des revendications précédentes, dans lequel les premier et second composants de logement (1, 2 ; 30, 20) sont réalisés sous la forme d'un support de cartouche pour une cartouche (9) renfermant un produit et/ou sous la forme d'un boîtier pour un mécanisme d'entraînement destiné à être accouplé de manière fonctionnelle à la cartouche.

10. Procédé d'assemblage d'un dispositif d'administration de médicament comprenant un premier composant de logement (1 ; 30) comportant un réceptacle (12) de forme ovale ou elliptique en coupe radiale et un second composant de logement (2 ; 20) comportant une partie emboîtable (11) de forme ovale ou elliptique en coupe radiale et pouvant être emboîté au moins partiellement dans ledit premier composant de logement (1 ; 30) le long d'une direction axiale (13), le procédé comprenant les étapes suivantes :
- emboîter au moins partiellement l'un dans l'autre la partie emboîtable (11) et le réceptacle (12) des premier et second composants de logement (1, 2 ; 30, 20) dans une direction axiale (13) et générer une force de pré-tension (10) dirigée radialement entre les premier et second composants de logement (1, 2 ; 30, 20) afin d'assurer un verrouillage réciproque des premier et second composants de logement (1, 2 ; 30, 20) dans une configuration de pré-assujettissement,
- raccorder mutuellement, de façon non séparable, les premier et second composants de logement (1, 2 ; 30, 20) par soudage au laser.

11. Procédé selon la revendication 10, dans lequel les premier et second composants de logement (1, 2 ; 30, 20) comprennent une partie emboîtable (11) et un réceptacle (12) correspondant, la dimension extérieure ou au moins un diamètre extérieur de la partie emboîtable (11) étant, au moins au niveau de certaines sections, supérieur(e) à une dimension intérieure correspondante du réceptacle (12) et les premier et second composants de logement (1, 2 ; 30, 20) devenant soumis à une force de pré-tension mutuelle dans la direction radiale par le biais d'une rotation des premier et second composants de logement (1, 2 ; 30, 20) l'un par rapport à l'autre dans une direction circonférentielle (16).
